# EUROPEAN PATENT APPLICATION

(11) **EP 3 309 636 A1**
(43) Date of publication of application: **18.04.2018**
(21) Application number: 16782695.7
(22) Date of filing: 21.04.2016
(51) Int. Cl.: G05B 19/4099, A61B 17/58

(54) **METHOD FOR PRODUCING AN OCCLUSIVE BARRIER FOR BONE REGENERATION AND OCCLUSIVE BARRIER OBTAINED BY MEANS OF SAID METHOD**

(30) Priority: 23.04.2015 ES 201500309
(71) Applicant: Lizarazo Rozo, Mauricio Alberto, 48901 Barakaldo (Vizcaya) (ES)
(72) Inventor: Lizarazo Rozo, Mauricio Alberto, 48901 Barakaldo (Vizcaya) (ES)
(74) Representative: Buceta Facorro, Luis
(86) International application number: PCT/ES2016/070289
(87) International publication number: WO 2016/170218

(57) **Abstract**

The present invention discloses a production method for producing an occlusive barrier (1) for bone regeneration, and the occlusive barrier (1) obtained by means of said method. The method comprises obtaining a tomography image, processing and digitizing the tomography image, reducing the noise of the tomography image and converting same into a three-dimensional CAD file, importing the three-dimensional CAD file into CAD modeling software, defining an area corresponding to the occlusive barrier (1), generating a surface corresponding to the occlusive barrier (1), converting the occlusive barrier (1) into layers by means of CAM software, printing the occlusive barrier (1) in titanium according to the layers, subjecting the occlusive barrier (1) to a thermal treatment and to a surface sand-blasting treatment, and examining the orifices (4), thicknesses and dimensions of the occlusive barrier (1) in an optical gauge.

## Description

### Field of the Art

The present invention relates to the industry dedicated to bone regeneration.

### State of the Art

Guided bone regeneration (GBR) is currently, and has been for years, the treatment of choice for regenerating the alveolar bone necessary for the placement of dental implants. Guided bone regeneration (GBR) can also be defined as a technique for bone regeneration by inhibiting soft tissue proliferation, by means of closing the defect with a membrane after filling the defect with bone grafts or fillers to maintain the space.

Bone grafts and fillers claim to have a mechanical and biological function. There is a complex relationship in the bone graft-host interface where several factors can take part in the correct incorporation or non-incorporation of the graft, including among others graft vascularization, preservation techniques, local factors, systemic factors and mechanical properties (depending on the type, size and shape of the graft used). A suitable bone volume for osseointegration is essential for implant treatments. One of the prominent components of the stomatognathic system is the alveolar bone, which is a tooth-dependent structure, as it is formed together with teeth, supports said teeth as they perform their function and disappears once the teeth are lost.

Filler or graft materials (biological bone defect-filling products) are described among materials used for bone regeneration; and these materials include, among others, autologous grafts, allogeneic materials, xenogeneic materials, bone substitutes, guided bone regeneration techniques and the use of bone morphogenetic proteins.

In this sense, the different materials used can act with at least one of these three known mechanisms or processes:
- Osteogenesis: Synthesis of new bone from cells derived from the graft or host. It requires cells that are capable of generating bone.
- Osteoinduction: A cell differentiation and recruitment process, modulated by growth factors derived from the matrix of the graft, the osteogenic activity of which is stimulated by bone mineral extraction.
- Osteoconduction: A process through which the material of the graft provides a physical material, structure or environment suitable for new bone apposition by means of a predictable pattern, determined by the biology of the graft and the mechanical environment of the host-graft interface.

The ideal bone grafts and fillers should give rise to these three processes, in addition to being biocompatible and providing mechanical stability. Biocompatibility can be defined where a material is considered compatible and only causes the desired or tolerable reactions in the living organism.

In order to attain any of the processes indicated above, bone grafts have been the object of studies for more than four decades. The different options that can be mentioned include, among others:
A. Autologous or autogenous grafts: They refer to bone obtained from the patient themselves and therefore have very little antigenic capacity. They are obtained from intraoral sites (the chin, maxillary tuberosity, ascending branch) that are used for small defects or extraoral sites (iliac crest, tibia or cranial vault) when a larger amount is required. Each approach is chosen depending on the type, size and shape of the bone cavity, and the clinical experience and preference of the professional.
B. Allogeneic grafts or allografts: They come from individuals of the same species, but are genetically different. They can be classified according to their processing as:
   - Frozen allografts
   - Lyophilized (freeze-dried) allograft
   - Lyophilized and demineralized allografts
   - Irradiated bone.

The allograft has the advantages of being available in significant amounts, in different shapes and sizes, not sacrificing the host structures and not involving donor site morbidity. The drawbacks are related to the quality of the regenerated bone tissue, which is not always predictable. They have to be processed to eliminate their antigenic capacity.
C. Heterologous grafts or xenografts: They are natural grafts coming from another (animal) species and containing natural minerals of bone. For example, bovine bone and coral derivatives (Ostrix, Osteogén, Bio-oss, Interpore).
D. Alloplastic or synthetic grafts: They come from synthetically manufactured materials. They can be found in a variety of shapes, sizes and textures. Biological responses of the bone will depend on the manufacturing techniques, crystallinity, porosity and degree of reabsorption.

These materials can be: ceramic materials; synthetic calcium phosphate (hydroxyapatite and tricalcium phosphate) being the most widely used, for example. Polymers: such as Bioplan, HTR. Bioactive glass ceramic: consisting of phosphate and calcium salts, and silicon and sodium salts (Biogass, pedioglass, Biogran).

All implantation materials must trigger a reaction that is as physiologically as possible with their surrounding tissues. It is fundamental to know the normal biological processes triggered in the regeneration and the physical, mechanical and biological characteristics typical of each material.

Currently, in order to use autologous grafts a surgical procedure must be performed on the donor site to obtain said grafts, with the subsequent risk of postoperative morbidity, infection, pain, hemorrhage, muscle weakness, neurological lesion, necrosis of the graft, among others; furthermore, the surgery time increases significantly and in some cases the amount of extracted graft many be insufficient.

In the current bone regeneration technique, the professional must manually preform the device to be implanted in the patient. Since the devices or barriers are preformed, there may be spaces that will allow soft tissue invasion and entry of bacteria, with the subsequent risk of infection, and therefore of the treatment failing.

The professional currently operates on the patient without any planning, which considerably limits the knowledge of the related surgical field.

In the current guided bone regeneration (GBR) technique, neoformation is expected for bone regeneration as the graft or filler used is reabsorbed or broken down, since their function is to maintain the space.

Depending on the bone material used as graft or filler, the time required for material reabsorption in current guided bone regeneration (GBR) conditions new tissue formation. For example, in the case of using Cerasorb® tricalcium phosphate (synthetic ceramic graft) average reabsorption time is from 24 to 36 months; and in the case of Bio-oss® (heterologous bovine bone graft), it is not reabsorbed as it is a ceramic material, a mixture between filler material and bone being formed over time.

Guided bone regeneration (GBR) also requires waiting for at least 12 to 24 months until the bone is eventually formed in order to be able to place the implants and leave them in for six more months for osseointegration, and then proceed with the dental rehabilitation of the patient.

The use of flexible membranes that collapse under their own weight in current guided bone regeneration (GBR) processes is the cause of the reduction in the required bone volume sought in said regeneration.

Koustopoulos stated in 2001 that "the mere fact of filling a space with a biomaterial adjacent to the bone does not necessarily increase bone formation, but on the contrary may inhibit it;" this demonstrates how unreliable the current technique is in alveolar bone regeneration, which is necessary and indispensable for implant placement.

### Object of the Invention

Breakthroughs in biotechnology have given rise to alternatives to fillers and bone grafts, such as a customized occlusive titanium barrier which is ideal due to its high osteoconductive capacity.

Occlusive barriers are comprised in the sector involving additive techniques, such as laser sintering, and subtractive techniques, such as computerized machining, that are applied in medical sciences such as odontology.

The occlusive barrier is therefore a computer-designed biomedical device that is custom made for the patient and produced by titanium laser sintering technology, said device being adapted to the measurement of the anatomical structure of the patient. The object of the occlusive barrier is to create a space between the bone tissue and gum tissue to promote bone growth from the stem cell layer that is covering the outer surface of the bone (endosteum). Its function therefore consists of maintaining the space in order to support a blood clot which ultimately leads to tissue regeneration. This biomedical device or structure allows keeping the blood clot stable and isolated from the external environment, furthermore preventing bacterial invasion which would prevent the regeneration process.

In this sense, the occlusive barrier is printed in one or more parts. When the occlusive barrier is formed by more than one part, said occlusive barrier will comprise a concavity in at least two of the parts, each of the concavities being configured to partially surround a tooth, such that the concavities jointly surround the tooth. Therefore, said parts are complementary to jointly define at least one through hole in which the tooth can be housed.

Biocompatibility is defined as the ability of a material to suitably respond to the host in a specific application. This type of material is known as a biomaterial used in medicine, in this case odontology, for interacting with biological systems by inducing a specific biological activity.

Titanium is considered the ideal biomaterial for making customized occlusive barriers for the following reasons:
- Titanium is inert; the oxide coat in contact with the tissues is insoluble so ions that may react with organic molecules are not released.
- Titanium in living tissues represents a surface on which bone grows and adheres to the metal.

### Description of the Drawings

Figure 1 shows an occlusive barrier object of the present invention according to a preferred embodiment.
Figure 2 shows the occlusive barrier object of the present invention according to another preferred embodiment.
Figure 3 shows another view of the occlusive barrier shown in Figure 1.
Figure 4 shows a section view of the occlusive barrier according to the preferred embodiment shown in Figures 1 and 3.
Figures 5 to 7 show the occlusive barrier object of the present invention according to another additional preferred embodiment.

### Detailed Description of the Invention

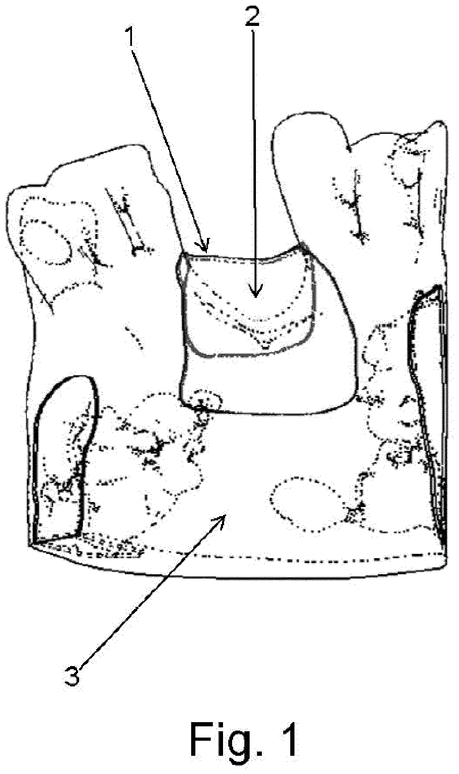

Figures 1 to 3 show an occlusive barrier (1) which is a customized biomedical device made of titanium that is personalized for each patient. The occlusive barrier (1) allows the reconstruction of new bone tissue and/or is intended for the replacement and regeneration of destroyed or lost structures, without having to use filler materials or bone grafts.

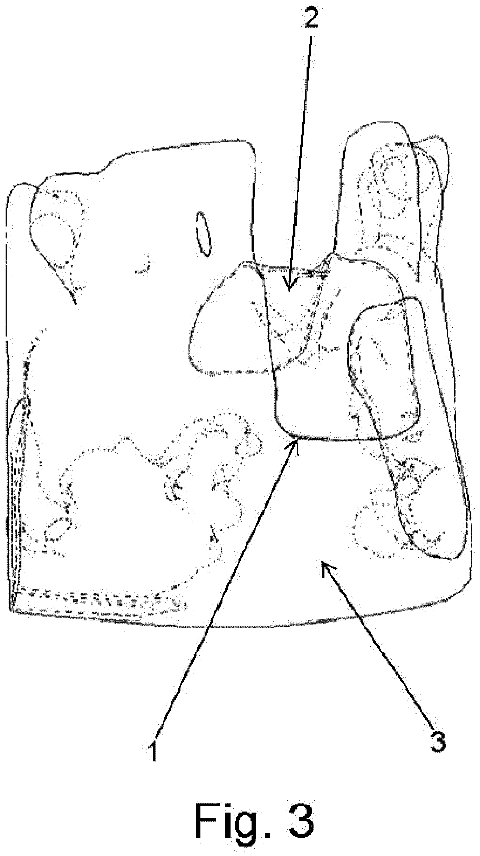

As a biomedical device, the customized occlusive barrier (1) induces bone neoformation by acting as a biological barrier to prevent the migration of connective tissue epithelial cells and/or of bacteria which would inhibit bone growth. The occlusive barrier (1) is 100% compatible, has an occlusive property as it is customized for the patient, since it completely adapts to the surgical site, having the capacity to maintain a total regenerative space (2) and the possibility of vascularization. The occlusive barrier (1) is designed to assure the three-dimensional reconstruction of defects of the alveolar bone (3) and to make restoration of the alveolar bone (3) easier by means of the suitable fixing of the restoration material. The occlusive barrier (1) promotes a suitable space (2) for the formation of a natural molding from fibrin, a bone tissue precursor, as can be seen in Figures 1 and 3.

The occlusive barrier (1) has the osteoconduction mechanism given that it provides a physical material, structure and environment triggering a three-dimensional growth of capillaries, perivascular tissue, and most importantly, the recruitment of mesenchymal stem cells in the region for subsequent differentiation into osteoblasts modulated by growth factors. This scaffolding allows new bone formation by means of a predictable pattern, determined by the biology and the thickness and height dimensions previously determined during design and approved by the professional.

The design and production of the occlusive barrier (1) is described below. The process of developing customized occlusive barriers (1) involves a series of technological and technical processes used starting from the moment of performing 3D diagnostic imaging until the delivery of the biomedical device.

First, a tomography image of the patient is obtained and sent to the design laboratory. The image is processed and digitized there to determine the area where intervention is required, reducing the noise and converting it into a three-dimensional file which can be entered into any CAD modeling software.

Once this CAD file has been obtained, it is imported into the modeling software where the file is located and drawn on to define the area of the occlusive barrier (1) and generate a surface that will be converted into the occlusive barrier (1). Once approved and corrected by the physicians, the occlusive barrier (1) is exported for production.

The exported design file is used for production and post-processing is performed on said file in CAM software, converting it into layers for printing in titanium. The occlusive barrier (1) is printed in layers that are 30 or 60 microns thick, and then progresses to a machining phase once it is printed.

In this phase, the occlusive barrier (1) is initially subjected to a thermal treatment to reduce molecular stress and to make the occlusive barrier (1) stronger and more ductile, and then subjected to a surface sand-blasting treatment which allows the occlusive barrier (1) to have optimum characteristics for osseointegration. A porosity to favor osteoconduction and blood vessel formation around the occlusive barrier (1) is obtained by means of the surface sand-blasting treatment. Therefore, the occlusive barrier (1) is obtained with an arithmetic mean roughness (Ra) of 9-12 µm and a mean roughness amplitude (Rz) of 40-80 µm.

Finally, the thicknesses, orifices (4) and dimensions in the occlusive barrier (1) are examined in an extremely precise optical gauge to assure that the occlusive barrier (1) has the initially defined geometric characteristics. From there, it progresses to a sterilization treatment to be packaged and sent to the customer.

The occlusive barrier (1) is subjected to an anodizing treatment. Said anodizing treatment allows cleaning both organic and inorganic residues from the surface, obtaining a better resistance against corrosion, reducing the release of titanium ions into the physiological medium, obtaining greater surface hardness, improving osteoconduction properties and obtaining a color that is similar to the color of the gums. The color is important in cases in which the occlusive barrier (1) is exposed after placement in the patient in order to reduce the visual impact it produces.

The occlusive barrier (1) is between 0.3 and 0.6 millimeters thick. Lower values prove to be an obstacle in the capacity to maintain the space (2) and higher values make it difficult for the patient to accept the occlusive barrier (1) in the corresponding fixing region.

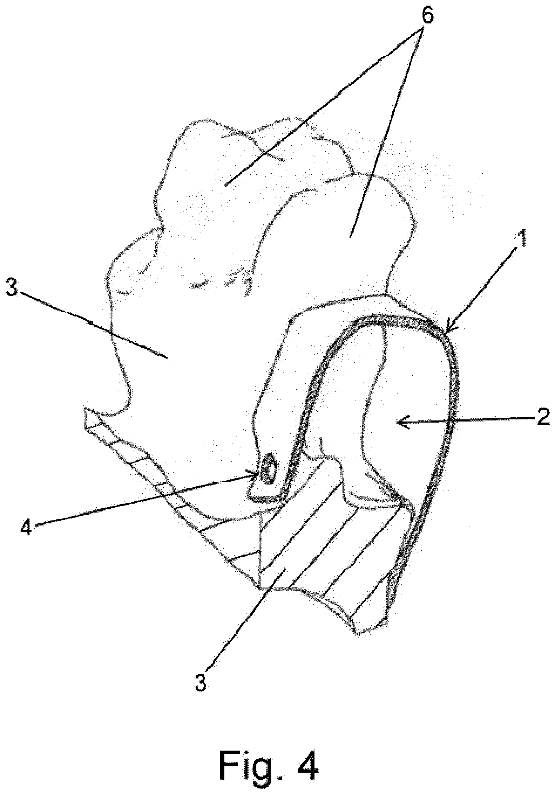

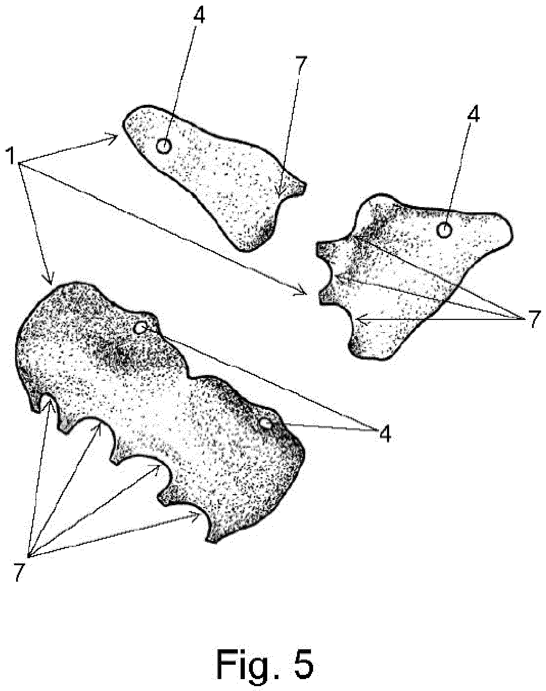

The orifices (4), which can be seen in Figures 4 and 5, are configured for the insertion of screws (5), preferably titanium screws. The orifices (4) are for fixing the occlusive barrier (1) to the alveolar bone (3) of the patient. For this reason, said orifices (4) are located in the occlusive barrier (1) depending on the patient, and more specifically on the available bone (3) of the patient.

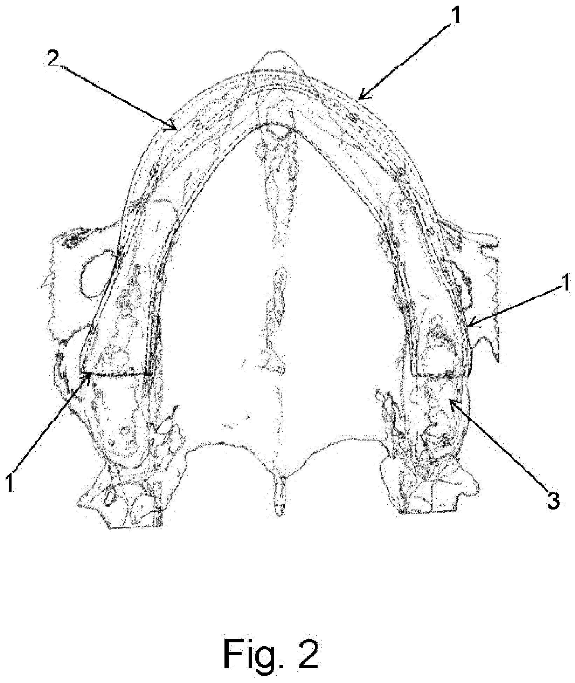

Occlusive barriers (1) can be partial or complete barriers. Occlusive barriers (1) are defined according to their longitudinal extension over the alveolar bone (3), where examples of partial occlusive barriers (1) can be seen in Figures 1 and 3 to 7 and an example of a complete occlusive barrier (1) can be seen in Figure 2. Therefore, partial occlusive barriers (1) can be limited by teeth (6) at least at one of the longitudinal ends thereof, whereas complete occlusive barriers (1) cover the entire longitudinal extension of the alveolar bone (3) in which the teeth (6) can be located and on which they are available. The longitudinal extension of the occlusive barriers (1) over the alveolar bone (3) can be greater than in the case of flexible membranes because the occlusive barriers (1) do not collapse under their own weight like said membranes do.

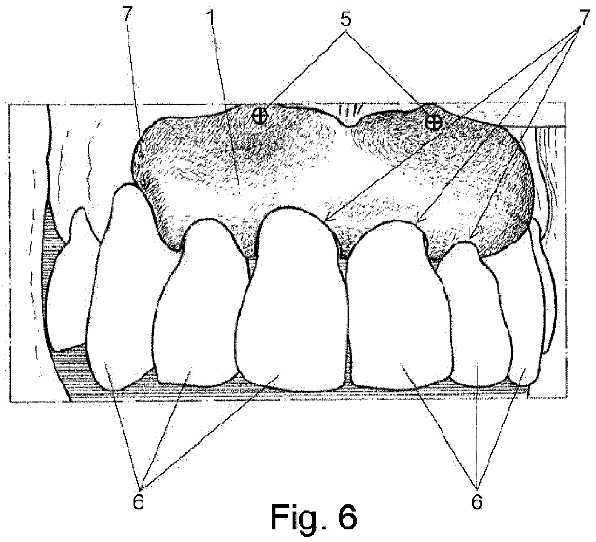

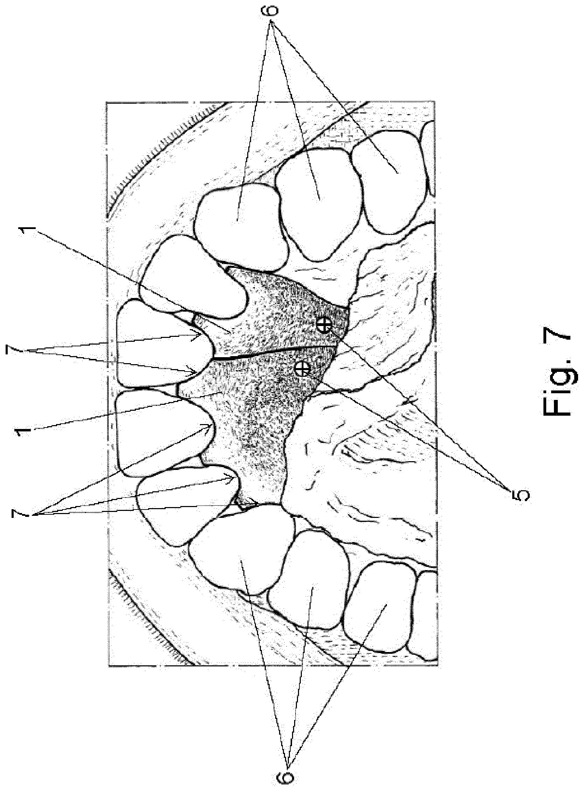

Likewise, according to the design and production described above, the occlusive barrier (1) can be printed in one part, as can be seen in Figures 1 to 3, or in several parts, as can be seen in Figures 5 to 7. Figure 5 shows the parts, specifically three in number, forming an example of the occlusive barrier (1), whereas Figures 6 and 7 together show the arrangement of said occlusive barrier (1).

In this sense, the occlusive barrier (1) is printed in one part or in at least two parts. When the occlusive barrier (1) is formed by more than one part, the occlusive barrier (1) will comprise a concavity (7) in at least two of the parts, each of the concavities (7) being configured to partially surround one of the teeth (6). In this case, the concavities (7) jointly define a through hole according to the outer contour of the tooth (6) for covering the bone (3) respecting the tooth (6). The occlusive barrier (1) is printed in several parts mainly due to the fact that the patient, despite experiencing a bone defect or wear, still sometimes have the tooth (6) or teeth (6) in the affected region. The present production method allows obtaining parts in the most optimum way according to each case.

The parts forming the occlusive barrier (1) can be obtained in complementary shapes in order to jointly cover the alveolar bone (3) completely or partially, leaving the space corresponding to the teeth (6) located in the region intended for receiving the occlusive barrier (1) free, furthermore leaving the corresponding gap (2) free. In the embodiment shown in Figures 5 to 7, the occlusive barrier (1) has three parts. As can be seen in Figure 7, the occlusive barrier (1) can also be formed by several parts to better adapt to the shape of the bone (3) and make the placement thereof easier, i.e., it can be formed by parts that are laterally complementary to one another to cover the affected region according to the longitudinal extension of the bone (3).

The titanium used for making the occlusive barrier (1) is a material designed to safely and effectively interact with biological systems. Biomaterial-host interactions do not pose any type of safety problem for the patient, i.e., it is 100% compatible. The titanium used is preferably a titanium alloy called Ti64 or Ti6Al4V, with a density of 4.43 g/c³.

Properties of medical titanium:
- Titanium is inert, the oxide coat in contact with the tissues is insoluble, so ions that may react with the organic molecules are not released.
- Titanium in living tissues represents a surface on which bone grows and adheres to the metal, forming an ankylotic anchor, also referred to as osseointegration. This reaction usually takes place in materials called bioactive materials and is the best base for functional dental implants.
- It has good mechanical properties; its tensile strength is very similar to that of stainless steel used in load-receiving surgical prostheses. It is much stronger than dentin or any bone cortex, allowing implants to withstand heavy loads.
- This metal is soft and malleable, which helps to absorb shock loading.

Titanium is a biocompatible metal (biomaterial) because body tissues tolerate its presence without any allergic reactions being observed in the immune system. This biocompatibility property of titanium, along with its mechanical characteristics such as hardness, lightness and strength, allow for a large number of medical applications, not only dental implants, but also hip and knee prostheses, bone screws, anti-trauma plates, components for the production of heart valves and pacemakers, surgical instruments, etc.

The occlusive barrier (1) therefore includes the following features:
- Cell occlusion: The occlusive barrier (1) has the property of being isolated from the gum tissue of the skin flap opened during surgery from the maturation of the fibrin blood clot in the space of the wound.
- Space maintenance capacity: The occlusive barrier (1) has the capacity to withstand collapsing determined by its rigidity. In other words, the occlusive barrier (1) has the physical property of having the capacity to withstand collapsing determined by its rigidity, assuring the bone volume previously determined in the design of the biomedical device.
- Tissue integration: The occlusive barrier (1) must be integrated, as much as possible, with the tissue where it is placed.

The present invention is characterized by requiring only one surgery on the site receiving the occlusive barrier (1), without needing any bone graft or filler. Since no filler whatsoever is required, the osteoconductive capacity of titanium allows blood vessels to construct scaffolding for osteogenic cells in the blood clot, providing conditions suitable for new bone growth.

Likewise, since no filler whatsoever is required, mechanisms for the reabsorption of foreign matter are not required from a biological perspective. Bone neoformation therefore starts once the occlusive barrier (1) is put in place, i.e., tissue regeneration time is much shorter.

The technology used in the biomedical device design and production process allows first knowing the anatomy of the surgical field in all three of its dimensions, even making virtual operation possible.

As described, the present invention describes the use of digitized design and manufacturing processes (CAD-CAM) and software necessary for printing the occlusive barrier (1) for the patient in a customized manner. Since the occlusive barrier (1) is a custom-made device, it adapts to the patient completely and allows for complete peripheral sealing, preventing the entry of soft tissue and bacteria, situations which allow assuring the success of the treatment. The present invention is characterized by the possibility of placing the implants when surgery for the occlusive barrier (1) is being performed, so that as it is formed, the new bone tissue will osseointegrate with the implants, leading to a very significant time savings to start patient rehabilitation.

The conditions of treating the tissue which has the occlusive barrier (1) customized for the patient, made of titanium for medical use, 100% treatment success can be assured as long as all the conditions of technological and scientific capacity for its implementation, in addition to the protocols requiring compulsory compliance necessary for obtaining the anticipated result, are complied with.

## Claims

1. A production method for producing an occlusive barrier (1) for bone regeneration, **characterized in that** it comprises the steps of:
- obtaining a tomography image;
- processing and digitizing the tomography image;
- reducing the noise of the tomography image;
- converting the tomography image into a three-dimensional CAD file;
- importing the three-dimensional CAD file into CAD modeling software;
- defining an area corresponding to the occlusive barrier (1) ;
- generating a surface corresponding to the occlusive barrier (1);
- converting the occlusive barrier (1) into layers by means of CAM software;
- printing the occlusive barrier (1) in titanium according to the layers;
- subjecting the occlusive barrier (1) to a thermal treatment;
- subjecting the occlusive barrier (1) to a surface sand-blasting treatment; and
- examining the orifices (4), thicknesses and dimensions of the occlusive barrier (1) in an optical gauge.

2. The production method according to claim 1, **characterized in that** the layers in which the occlusive barrier (1) is printed in titanium have a thickness of 30 or 60 microns.

3. The production method according to claim 1 or 2, **characterized in that** it additionally comprises subjecting the occlusive barrier (1) to a sterilization treatment.

4. The production method according to any one of the preceding claims, **characterized in that** the occlusive barrier (1) is printed in one part.

5. The production method according to any one of claims 1 to 3, **characterized in that** the occlusive barrier (1) is printed in at least two parts.

6. The production method according to claim 5, **characterized in that** the parts are complementary to jointly define at least one through hole in which a tooth (6) can be housed.

7. An occlusive barrier (1) for bone regeneration obtained by means of the production method according to any one of the preceding claims.
